Europäisches Patentamt

European Patent Office

Office européen des brevets

(19)

(11) Publication number: **0 166 263**
**A1**

# EUROPEAN PATENT APPLICATION

(21) Application number: **85106694.4**

(22) Date of filing: **30.05.85**

(51) Int. Cl.⁴: **A 61 L 27/00**, A 61 L 25/00

(30) Priority: **31.05.84 JP 109693/84**
**31.05.84 JP 109694/84**

(43) Date of publication of application: **02.01.86**
**Bulletin 86/1**

(84) Designated Contracting States: **BE DE FR GB NL SE**

(71) Applicant: **THE GREEN CROSS CORPORATION, 15-1,**
**Imabashi-1-chome Higashi-ku Osaka-shi,**
**Osaka 541 (JP)**
Applicant: **MITSUBISHI MINING & CEMENT CO., LTD.,**
**5-1, Marunouchi 1-chome Chiyoda-ku, Tokyo 100 (JP)**

(72) Inventor: **Kurosawa, Hisashi, 3-9-9-912, Nishikasai**
**Edogawa-ku, Tokyo (JP)**
Inventor: **Murase, Kenichi, 6-10-13-401, Nishikasai**
**Edogawa-ku, Tokyo (JP)**
Inventor: **Nishimaki, Hideo, 2-3-6, Akai, Daito-shi Osaka**
**(JP)**
Inventor: **Kitagawa, Shuzo, 56, Kitafunebashi-cho,**
**Hirakata-shi Osaka (JP)**
Inventor: **Nishida, Masayuki, 2-12-1, Kayogaoka,**
**Nagaokakyo-shi Kyoto (JP)**
Inventor: **Ono, Mikiya, 382-1, Ooaza Nakayama,**
**Hanno-shi Saitama-ken (JP)**
Inventor: **Takeuchi, Hiroyasu, 1019, Ooaza Yokoze**
**Yokozemura, Chichibu-gun Saitama-ken (JP)**
Inventor: **Ohkubo, Yoshitaka, 396, Ooaza Kuna,**
**Chichibu-shi Saitama-ken (JP)**

(74) Representative: **Strehl, Schübel-Hopf, Schulz,**
**Widenmayerstrasse 17 Postfach 22 03 45,**
**D-8000 München 22 (DE)**

(54) Filler composition for filling in defect or hollow portion of bone and kit or set for the preparation of the filler composition.

(57) According to the present invention, a filler composition is provided. The filler composition is filled in a defect or hollow portion of bone. The filler composition comprises fibrin and a calcium phosphate compound. The present invention also provides a kit or set for the preparation of the filler composition. The kit or set comprises at least two containers. One container includes fibrinogen and the other container includes thrombin. The kit or set further contains a calcium phosphate compound and calcium chloride.

EP 0 166 263 A1

Filler Composition for Filling In Defect or Hollow Portion of Bone and Kit or Set for the Preparation of the Filler Composition.

## BACKGROUND OF THE INVENTION:

Field of the Invention;

The present invention relates to a filler composition for filling in a defect or hollow portion of bone and containing fibrin and a calcium phosphate compound and a kit or set for preparing such a filler composition.

Related Art Statement;

In the surgical and orthopedic treatments, prosthesis operations are often required for filling in defects or hollow portions of bone resulting from serious fracture of bone or surgical removal of bone tumor. Also in the dental treatment, prosthesis operations are frequently required for filling in injured portions formed in alveolar bone caused by pyorrhea alveolaris. To cope with such a case, it has been a common practice to resect flank bone and the like from the patient to fill up in the defect or hollow portion of bone, thereby to promote early remedy of the bone tissue. However, by means of such an operation, normal bone tissue must be picked up from an unspoilt portion to cause additional pains to the patient in addition to a great deal of troubles in operation. Moreover, when the volume of defect or void in the patient's bone is large, the amount of bone obtainable from his own body is not always sufficient for fully filling in the defect or void. In such a case, it is inevitable to use a substituent for the patient's own bone tissue.

The substituting materials for such purpose include, for instance homogeneous or heterogeneous bone. However, both of the aforementioned homogeneous and heterogeneous bones might cause foreign body reaction or other problems, and the post-operation course after the implantation of such bone is not always satisfactory. For this reason, implantation of such substituting materials has not yet been applied for practical operation.

On the other hand, a variety of metallic and organic materials have hitherto been used as the substituent for hard tissues in the living body. However, it has been recognized that these materials have tendencies to be dissolved or otherwise deteriorated in the environment of living tissue or to be toxic to the living body, and that they cause so-called foreign body reactions. Ceramic materials attracted public attention, since they are excellent in compatibility with living tissues. Recently proposed are an artificial bone, an artificial joint or an artificial tooth made of monocrystal or sintered products of alumina, carbon, tricalcium phosphate or hydroxyapatite.

It has been tried to fill in a defect or void of bone with such a sintered product or monocrystal of ceramic materials.

However, any of the preceding trials has not yet been in practical use, since particular defect of bone, which requires treatment or filling, has a complicated shape different individually to call for a sintered mass or monocrystalline implant material having a shape and dimension well agreed with those of the defect. It is difficult to prepare such a sintered or monocrystalline implant material by machining. In addition, even when a sintered or monocrystalline mass is implanted in such a defect, absorption of bone occurs in the vicinity of the implanted material due to stimulus caused by the implanted foreign matter, leading to loosening or similar problems. For these reasons, use of sintered or monocrystalline implant material has not yet reached to the level of practical application.

It has been conceived to prepare a porous implant by machining a sintered material or molding powders added with flammable fibers before sintering to form pores at the subsequent sintering step. However, the porosity of the thus prepared porous implant cannot be increased so high, in a case where a ceramic sintered mass is machined to form voids therein, since ceramics are generally fragile to be easily broken during the machining step. In a case where a porous implant material is prepared by the process wherein starting powders of ceramic material are mixed with flammable fibers followed by molding and sintering, increase in porosity of the finished product is limited by the difficulty that the amount of fibers which can be mixed with the starting material powders is limited. Since the porosity of any of the sintered ceramic material prepared through either one of the processes mentioned above could not be increased so high as required for allowing the bone forming tissues of the living body to penetrate deeply into the implant material, relatively long time was spent until an integral structure had been formed by the implanted mass.

OBJECTS AND SUMMARY OF THE INVENTION:

Accordingly, a primary object of this invention is to provide a filler composition to be filled in a defect or hollow portion of bone and a kit or set for the preparation thereof, which composition is particularly improved in compatibility with the living body, causes little foreign body reaction and can form new bone tissue within a very short period of time, the filler per se being absorbed in and then being substituted by living body or tissue so as to make it possible to form new bone tissue throughout the filler composition up to the center portion thereof.

Another object of this invention is to provide a filler composition to be filled in a defect or hollow portion of bone and a kit or set for the preparation thereof, which composition accelerates formation of bone tissue in the filled portion to recover and remedy the structure and function at and near the defects of bone tissue .

A further object of this invention is to provide a filler composition to be filled in a defect or hollow portion of bone and a kit or set for the preparation thereof, by the use of which new bone is formed very rapidly.

A still further object of this invention is to provide a filler composition to be filled in a defect or hollow portion of bone and a kit or set for the preparation thereof, which can be easily molded to have a shape fitted snugly in a particular void to be filled therewith.

The above and other objects of the invention will be fully understood from the following description.

The filler composition to be filled in a defect or hollow portion of bone, according to the invention, comprises fibrin and a calcium phosphate compound. Also provided by the invention is a kit or set for the preparation of the filler composition to be filled in a defect or hollow portion of bone, comprising at least two containers, one container including

0166263

- 6 -

fibrinogen and the other including thrombin, said kit or set further containing a calcium phosphate compound and calcium chloride.

DESCRIPTION OF THE INVENTION:

The present invention will now be described in detail.

At the initial stage of development, we, the inventors, have turned our attention to the fact that new bone is formed at a portion of a defect or void of bone in which a calcium phosphate compound is filled. Based on the obserbed fact, we have intended to utilize the bone forming capability of a calcium phosphate compound. Calcium phosphate compounds, which may be used in the invention, include tricalcium phosphate, hydroxyapatite, tetracalcium phosphate, oxyapatite, calcium pyrophosphate, fluoroapatite, hydroxyapatite derivatives having hydroxyl groups substituted by fluorine ions, and mixtures thereof. Of these, it is preferred to select the compounds accelerating formation of new bone such as tricalcium phosphate, hydroxyapatite, fluoroapatite or tetracalcium phosphate or mixtures thereof. Amongst them, hydroxyapatite is the most preferred, since it facilitates formation of new bone at the highest rate. In order to further accelerate formation of new bone, it is desirable to use hydroxyapatite subjected to heat treatment at a temperature of higher than 500 ℃, preferably at a temperature of higher than 700℃. The only

parameter limiting the upper heat treatment temperature is the decomposition of hydroxyapatite. Calcium phosphate compounds used in this invention may be artificially synthesized through the known wet processes, dry processes and hydrothermal processes, and calcium phosphate compounds obtainable from natural resources, such as bone, may also be conveniently used.

On the other hand, fibrin used in the composition of the invention is prepared from fibrinogen originated from human or animal plasma. In consideration of the compatibility with the living tissue in which the filler composition is filled, it is preferable to use fibrinogen of human origin when the filler composition is applied for human being, and to use fibrinogen of animal origin when the filler composition is applied for an animal. Dried fibrinogens for medical use prepared in accordance with the "Standard for Medicines Prepared through Biological Preparation Processes" edited under supervision of Ministry of Health and Welfare, Pharmaceutical and Supply Bureau, pages 201 to 203 (1979), may be used as the starting fibrinogen materials in this invention, an example of commercially available product being a powder-form product supplied by The Green Cross Corporation under the Trade Name "Fibrinogen-Green Cross". This commercially available product is composed of dried fibrinogen added with a coagulative protein and stabilizers including sodium citrate and a monosaccharide such as glucose, fructose and mannitol, and is dissolved, prior to use, in distilled water for injection or in a buffer solution having a pH value of 6 to 7 and a low salt content. A 0.01 to 0.03 mol citric acid buffer solution may be conveniently used for this purpose. Fibrinogen may be dissolved at a temperature of from 32℃ to 36℃, and it is preferred to maintain the pressure in a phial containing the fibrinogen at a reduced pressure. Under such a condition, the dried fibrinogen powder is dissolved to form a solution of about 2 to 10 g of fibrinogen powder in 100 mℓ of distilled water or buffer solution.

Fibrinogen may be used in the powder form. As will be described in detail hereinafter, fibrinogen is solidified during the step of preparing the filler composition of the invention by the action of thrombin and calcium chloride added as the solidifying agents to be converted into fibrin. It is conveninent to use the kit or set defined in the appended claims in preparation of the filler composition.

In preparation of the filler composition to be filled in a defect or hollow portion of bone, according to the invention, the aforementioned calcium phosphate compounds may be used in a powder or particle form (hereinafter inclusively referred to as powder-particle form) or in a granulated form, or may be molded into a porous mass of calcium phosphate compounds.

When any one or a mixture of calcium phosphate compounds is used in the powder-particle form, the deposit or precipitate obtained by, for example a wet process is separated from the solution by means of filtration or centrifugal separation, followed by drying, and then being pulverized to prepare a powder-particle form product. The powder-particle form calcium phosphate compounds prepared through dry and hydrothermal processes may be pulverized, as desired. Similarly, calcium phosphate compounds of natural origin such as bone are processed through pulverization steps. It is preferable to use powders or particles each having a particle size of up to 5 mm for the preparation of the filler composition of the invention.

The thus prepared calcium phosphate compounds of powder-particle form may be used directly in the filler composition of the invention, or the kit or set for the preparation of the same may be used without any further processing. However, in order to improve the crystal formation property of the particles thereby to improve compatibility with living body, to prevent infection of bactera contained in the particle, and to subject the particles to sufficient thermal sterilization thereby to preclude rejection reaction caused by the contained organic substances, it is desirous that the calcium phosphate compounds prepared through the wet processes be baked at a temperature of higher than 500°C, preferably higher than 700°C, followed by pulverization, if necessary, to obtain powder-particle form materials.

Any of the thus prepared powder-particle form calcium phosphate compounds obtained through respective synthetic processes or from natural origins, or those processed through additional baking treatment, may be added with a liquid, such as water or isotonic sodium chloride solution, and then granulated using a rolling type granulator. The diameter of the thus prepared granules is not limited, and preferably ranges within about 0.1 to 5 mm for facilitating preparation of the filler composition of the invention and for preventing the granules contained in the composition from flowing out of the filled region.

Anyway, it is convenient to prepare a mixture of a solution or powder of fibrinogen and one or more of the powder-particle form or granule form calcium phosphate compounds for the preparation of the filler composition of the invention. Separately, in a preferred embodiment, a mixed solution of thrombin and calcium chloride is prepared by dissolving them in an isotonic sodium chloride solution or a citric acid buffer solution and is used as a coagulant or solidifier. In general, it is preferred that the content of thrombin ranges within 1 to 500 NIH units/ml and the content of calcium chloride ranges within 10 to 100 millimols/ml. If the

contents of thrombin and calcium chloride are less than the lower limits, fibrinogen is not solidified satisfactorily, whereas the coagulation or solidification action is not further enhanced even if the content of the coagulative mixed solution is increased in excess of the upper limit.

In a case where the filler composition of the invention is filled in a certain region in a living bone, an inhibitor for proteinase may be added. Such an inhibitor may preferably be included in the mixed solution described hereinabove. Preferable inhibitors for proteinase, which may be added to the filler composition of the invention, include inhibitors for plasmine, and specific examples thereof are aprotinin, ε-aminocaproic acid, tranexamic acid and soybean trypsin inhibitor. Such an inhibitor for proteinase may be added, in general, in an amount of about 100 to 5000 KIE units/mℓ.

Upon mixing and stirring the mixture of fibrinogen and the calcium phosphate compound with the latter-mentioned coagulative mixed solution, fibrinogen is coagulated to be converted into fibrin, which is insoluble in water, by the action of thrombin and calcium chloride within about 10 to 30 minutes. As the result of the coagulation or solidification reaction, the filler composition to be filled in a defect or hollow portion of bone, according to the present invention, is prepared wherein a powder-particle form or granular calcium phosphate compound is dispersed in gluey fibrin.

Since a reaction time ranging within about 10 to 30 minutes is required for the coagulation or solidification reaction between fibrinogen and the mixed coagulative solution containing thrombin and calcium chloride, the filler composition of the invention to be filled in a defect or hollow portion of bone may be prepared by adding the mixed coagulative solution containing thrombin and calcium chloride to a solution of fibrinogen or powder-form fibrinogen, followed by admixing with a powder-particle form or granular calcium phosphate compound under agitation.

The kit or set for the preparation of the filler composition to be filled in a defect or hollow portion of bone, according to the invention, may be provided in any combinations, other than the combinations where thrombin and fibrinogen are contained in a same container. Examples of preferred combinations will be set forth as follows:

(a)  A combination kit comprising a first container containing a calcium phosphate compound and a powder or solution of fibrinogen, and a second container containing thrombin and calcium chloride optionally added with an inhibitor for proteinase;

(b)  A combination kit comprising a first container

0166263

- 13 -

containing a calcium phosphate compound, a second container
containing a powder or solution of fibrinogen, and a third
container containing thrombin and calcium chloride optionally
added with an inhibitor for proteinase;  and

(c)    A combination kit comprising a first container
containing a calcium phosphate compound, a second container
containing a solution or powder of fibrinogen, a third
container containing thrombin, a fourth container containing
calcium chloride, and a fifth container containing an
inhibitor for proteinase.

As has been described hereinbefore, the filler
composition to be filled in a defect or hollow portion of bone,
according to the present invention, may be prepared by using
a porous mass of a calcium phosphate compound.  When a porous
mass of a calcium phosphate compound is used, the aforementioned
powder-form calcium phosphate compound is suspended in a liquid,
such as distilled water, to form a slurry which is impregnated
in continuous pores of an organic material having a spongy
structure through which continuous pores passes to form
three-dimensional channels.  Then, the sponge of organic
material impregnated with the slurry is dried and heated to
a sufficiently high temperature so that the organic material is
burnt off, whereby a porous mass of calcium phosphate compound
having continuous channels of three-dimensional network structure is prepared. It should be easily understood that continuous channels having a three-dimensional network structure corresponding to the already burnt-off body or solid portions of the organic material pass throughout the thus prepared porous mass of the calcium phosphate compound. Thereafter, the porous mass of the calcium phosphate compound is dipped in a solution of fibrinogen or a powder of fibrinogen and then a solution of the aforementioned coagulative mixture containing thrombin and calcium chloride optionally added with an inhibitor for proteinase is dropped over the porous mass of calcium phosphate compound containing fibrinogen to coagulate and solidify the fibrinogen, whereupon another embodiment of the filler composition according to the invention is prepared, wherein fibrin is filled in continuous pores or channels passing through the porous mass of calcium phosphate compound. Since the reaction of fibrinogen with the coagulative mixture including thrombin and calcium chloride requires a certain time period, as decribed before, a mixture containing thrombin and calcium chloride may be admixed with a solution or powder of fibrinogen followed by immediate dipping of the porous mass of calcium phosphate compound in the admixed solution, or the admixed solution may be dropped over the porous mass to prepare the filler composition of the present invention.

Irrespective of whether a calcium phosphate compound is used in the powder-particle form, granular form or in the form of porous mass, the calcium phosphate compound may be initially filled in a defect or hollow portion of bone and then the admixture of the aforementioned coagulative mixture admixed with a powder or solution of fibrinogen is poured into the defect or void of bone now charged with the calcium phosphate compound, or alternatively the admixture of the aforementioned coagulative mixture admixed with a powder or solution of fibrinogen is initially poured into a defect or void of bone followed by filling in the calcium phosphate compound.

It is preferred that the filler composition according to the invention contains 5 to 95 vol% of a calcium phosphate compound and the balance of fibrin. If the content of calcium phosphate compound is less than 5 vol%, formation of new bone might be retarded. On the contrary, if the content of calcium phosphate compound exceeds 95 vol% with attendant decrease in content of fibrin, formation of new blood vessel is adversely affected.

Calcium phosphate compounds have functions to promote or facilitate formation of new bone when they are filled in a defect or hollow portion of bone. On the other hand, fibrin is a hard protein formed by the interaction between fibrinogen and thrombin and has an agglutinating function to join end faces of wounded portions. Due to this aggulutinating function, fibroblasts are formed on the wounded surfaces and the fibroblasts are converted into fibrous cells to be fixed as new tissues with formation of new blood vessels. By the use of the filler composition of the invention, the calcium phosphate compound is retained by the agglutinating function of fibrin at a fixed location for allowing the calcium phosphate compound to exert its function of promoting formation of new bone and concurrently formation of new blood vessels is also accelerated by fibrin contained in the filler composition, so that repair or remedy of living tissue is remarkably accelerated. Particular advantage obtainable by the use of the filler composition to be filled in a defect or hollow portion of bone, according to the invention, is that new blood vessels are formed and extend through the filler composition and reach the center thereof to accelerate formation of new bone tissue.

The filler composition of the invention may be stored under a germ-free condition in an isotonic sodium chloride solution or in a buffer solution having a pH value of from 6 to 7 and lower contents of the component salts, such as a 0.01 to 0.03 M citric acid buffer solution, and may be cut into a segment having a shape and dimension agreed with those of a defect in the bone followed by filling the segment in the void portion in the bone. Alternatively, the thus stored filler composition may be cut into small pieces, for example, of about 1 mm to 5 cm and a plurality of such small cut pieces are filled in a defect or hollow portion of bone.

EXAMPLES OF THE INVENTION:

The present invention will now be described more specifically with reference to the examples thereof.

EXAMPLE 1

Using a kit of the invention, 60 mg of fibrinogen extracted from the blood of a rabbit followed by refinement was dissolved in a citric acid buffer solution (pH 6) to prepare a 9% solution, to which added was 1 mℓ of a 40 mM calcium chloride solution containing 4 NIH units/mℓ of thrombin and 3000 KIE units/mℓ of aprotinin to prepare a composition (hereinafter referred to as "fibrin composition"). The thus prepared "fibrin composition" was mixed , respectively, with a hydroxyapatite powder (calcined at 900℃), a tricalcium phosphate powder (calcined at 1150℃) and a tetracalcium phosphate powder (calcined at 1350℃) in a mixing ratio by volume of 1 : 1 to be solidified, whereby the filler compositions to be filled in defects and hollow portions of bone were prepared. Each of the thus prepared filler compositions was cut to prepare a segment having the dimensions of 5mm×4mm×3mm, and the segment was put into a 0.01 M citric acid buffer solution.

A pore or void having dimensions of 5mm×4mm×3mm was

0166263

- 18 -

formed in a femur of each rabbit, and each of the samples of the filler compositions of the invention was filled in the thus formed pore of each rabbit. The progress of repair after four weeks was observed. Similar pores were cut in femurs of other rabbits, and filled with comparison samples composed, respectively, of merely the "fibrin composition", merely the hydroxyapatite powder, merely the tricalcium phosphate powder and merely the tetracalcium phosphate powder. Likewise, the progress of repair after four weeks of each of the rabbits filled with the aforementioned comparison sample fillers was observed. As a control, a femur of a rabbit is cut to form a pore having the same dimensions but left without filling any filler. The progress of repair after four weeks was observed similarly.

The results showed that new bones were grown even at the center of the artificially cut off pores when the filler compositions prepared from the kits of the invention were filled in the pores. Particularly, the best result was obtained by the use of the combination of the "fibrin composition" and hydroxyapatite in that growth extent of new bone was the greatest.

On the contrary, in the comparison sample filled merely with the "fibrin composition" and in another comparison sample having the pore filled with no filler composition, the artificially cut off defects, <u>i.e.</u> pores, were filled with soft texture and new bone tissues were observed only at the inner peripheries of the defects.

In the comparison samples where only hydroxyapatite, tricalcium phosphate and tetracalcium phosphate were used, growth of new bone could scarcely appreciable at the center of the pores although some extents of grown new bone tissue were observed internally of the filler compositions,

## EXAMPLE 2

The "fibrin composition" was mixed with a powder of hydroxyapatite (calcined at 1200°C) in the mixing ratios, respectively, of 40 to 1, 20 to 1, 1 to 1, 1 to 20 and 1 to 40, by volume. Each of the mixtures was solidified similarly in Example 1, and the solidified mass was cut into samples each having dimensions of 5×4×3 mm which were dipped in the same liquids as described in Example 1 to prepare sample filler materials. Respective sample filler materials were then filled in pores formed in femurs of rabbits, each having dimensions of 5×4×3 mm. The progresses of remedy course were observed after 4 weeks.

The results were that new bone tissues were grown to extend onto the centers of pores in which fillers prepared from the mixtures containing the "fibrin composition" and hydroxyapatite in the ratios, by volume, of 1 to 20, 1 to 1

0166263

- 20 -

and 20 to 1, and that new bone tissues did not extend onto the centers of pores in which fillers prepared from the mixtures containing the "fibrin composition" and hydroxyapatite in the ratios, by volume, of 1 to 40 and 40 to 1.

EXAMPLE 3

A fibrinogen of human origin was used to prepare a "fibrin composition" similarly in Example 1. The thus prepared "fibrin composition" was mixed with hydroxyapatite (calcined at 800℃ and granulated and having a particle size of 2.0 to 1.0 mm in diameter) to prepare a mixture of the "fibrin composition" and hydroxyapatite mixed in a ratio of 1 to 10 (by volume). The mixture was coagulated or solidified, and the solidified mass was cut into a piece of $5 \times 5 \times 5$ mm to prepare a sample filler composition which was dipped in the same liquids as used in Example 1.

A bone tumor formed in a femur of a human being was resected and the thus formed void was filled in the sample filler composition described above, and the progress of remedy course after the operation was observed by means of roentgenogram and through scintigram. The observation through the roentgenogram revealed that formation of new bone was recognized after one week from the operation, and that new bone was growing thereafter so that the boundary between the filled sample and the thus grown bone tissue became vague after 4 weeks. On the other hand, the observation through the scintigram showed that the portion impaired by the tumor in cold condition was changed to be in hot condition after the operation to indicate that formation of new bone was prosperous.

## EXAMPLE 4

A porous hydroxyapatite mass (calcined at 1200°C and having a porosity of 80%) was dipped in a solution containing the same fibrinogen as used in Example 1. After picked up from the solution, a calcium chloride solution added with thrombin and aprotinin, which was the same as used in Example 1, was dropped to the porous mass, whereby a filler composition of the invention was prepared. The filler composition for filling in a defect or hollow portion of bone, prepared as aforementioned, was cut into a 5×4×3 mm sample and then put into a 0.01 M citric acid buffer solution.

A 5×4×3 mm pore was formed in a femur of a rabbit, and the pore was filled with the aforementioned sample filler. The progress of remedy course was observed after 4 weeks to recognize that new bone tissue had been grown to extend onto the center of the filled sample.

- 22 -

CLAIMS

1.      A filler composition to be filled in a defect or hollow portion of bone comprising fibrin and a calcium phosphate compound.

2.      The composition according to claim 1, wherein said composition contains 5 to 95 vol% of said fibrin and 95 to 5 vol% of said calcium phosphate compound.

3.      The composition according to claim 1 or 2, wherein said calcium phosphate compound is selected from the group consisting of tricalcium phosphate, hydroxyapatite, tetracalcium phosphate, oxyapatite, calcium pyrophosphate, fluoroapatite, hydroxyapatite derivatives having hydroxyl groups substituted by fluorine ions, and mixtures thereof.

4.      The composition according to any of the claims 1 to 3, wherein said calcium phosphate compound is in powder, particle form or in granule form and said powder, particle or granule is dispersed in fibrin.

5.      The composition according to claim 4, wherein said powder, particle or granule is a baked material.

6.      The composition according to any of the claims 1 to 3, wherein said calcium phosphate compound is in the form of porous mass having a multiplicity of continuous pores or channels of three-dimensional network structure and wherein said fibrin is filled in said pores or channels.

7.      The composition according to any of the claims 1 to 6, wherein said composition is stored in a liquid selected from the group consisting of isotonic sodium chloride solution and buffer solutions of low salt concentration.

8.      The composition according to any of the claims 1 to 7, wherein said fibrin is prepared by adding a coagulant or solidifier including thrombin and calcium chloride to fibrinogen.

9.      The composition according to any of the claims 1 to 8, wherein said composition contains an inhibitor for proteinase, which preferably is selected from the group consisting of aprotinin, ε-aminocaproic acid, tranexamic acid and soybean trypsin inhibitor.

10.      A kit or set for the preparation of a filler composition to be filled in a defect or hollow portion of bone, said kit or set comprising at least two containers, one container including fibrinogen and the other including thrombin, said kit or set further containing a calcium phosphate compound and calcium chloride.

11.      The kit or set according to claim 10, wherein said calcium phosphate compound is selected from the group consisting of tricalcium phosphate, hydroxyapatite, tetracalcium phosphate, oxyapatite, calcium pyrophosphate, fluoroapatite, hydroxyapatite derivatives having hydroxyl groups substituted by fluorine ions, and mixtures thereof.

0166263

- 24 -

12.    The kit or set according to claim 10 or 11, wherein said calcium phosphate compound is in powder, particle or granule form.

13.    The kit or set according to claim 12, wherein said powder, particles or granules is a baked material.

14.    The kit or set according to claim 10 or 11, wherein said calcium phosphate compound is in the form of porous mass having a multiplicity of continuous pores or channels of three-dimensional network structure.

15.    The kit or set according to any of the claims 10 to 14, wherein said fibrinogen is dried powder form fibrinogen, or is a fibrinogen solution.

16.    The kit or set according to claim 15, wherein said fibrinogen solution is formed by dissolving fibrinogen in a liquid selected from the group consisting of distilled water and buffer solutions of low salt concentration.

17.    The kit or set according to any of the claims 10 to 16, further comprising an inhibitor for proteinase, which preferably is selected from the group consisting of aprotinin, $\varepsilon$ -aminocaproic acid, tranexamic acid and soybean trypsin inhibitor.

18. The kit or set according to any of the claims 10 to 16, wherein said thrombin and said calcium chloride are dissolved in a liquid to form a mixed solution and said liquid is selected from the group consisting of isotonic sodium chloride solution and citric acid buffer solutions.

19. The kit or set according to claim 18, wherein said mixed solution contains 1 to 500 NIH units/ml of thrombin and 10 to 100 millimols/ml of calcium chloride.

20. The kit or set according to claim 18 or 19, wherein said mixed solution further contains an inhibitor for proteinase.

21. The kit or set according to claim 20, wherein the content of said inhibitor for said proteinase in said mixed solution ranges within 100 to 5000 KIE units/ml.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 068 149 (SERAPHARM)<br><br>* Page 10, lines 1-5; page 27, example 6; claims 1,6,10-12 * | 1,2,7-9 | A 61 L 27/00<br>A 61 L 25/00 |
| Y | | 3-6,10-21 | |
| X | FR-A-2 350 826 (BATTELLE-INSTITUT E.V.)<br>* Page 2, line 36; example 7; claims 1-5,8-10 * | 1,2 | |
| Y | | 4-6,10 12-21 | |
| X | EP-A-0 030 583 (OSTEO AG)<br>* Page 4, lines 18-23; claims 1,2 * | 1 | TECHNICAL FIELDS SEARCHED (Int. Cl.4) |
| Y | US-A-4 416 814 (O.A. BATTISTA)<br>* Column 6, lines 65-68 * | 3,11 | A 61 L |
| A | US-A-3 523 807 (M. GERENDAS) | 1 | |
| A | FR-A-2 410 477 (ETHICON, INC.)<br>* Claims 1,10 * | 1 | |
| A | EP-A-0 068 047 (SERAPHARM)<br>* Claims 1,4,11 * | 1 | |
| | --- -/- | | |

The present search report has been drawn up for all claims

| Place of search | Date of completion of the search | Examiner |
|---|---|---|
| THE HAGUE | 27-09-1985 | PELTRE CHR. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503. 03.82

European Patent Office

**EUROPEAN SEARCH REPORT**

| | **DOCUMENTS CONSIDERED TO BE RELEVANT** | | Page 2 |
|---|---|---|---|
| Category | Citation of document with indication, where appropriate, of relevant passages | Relevant to claim | CLASSIFICATION OF THE APPLICATION (Int. Cl.4) |
| A | FR-A-2 448 900 (IMMUNO AG)<br>* Page 3 *<br><br>----- | 1 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 27-09-1985 | PELTRE CHR. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82